(19) Europäisches Patentamt
European Patent Office
Office européen des brevets

(11) **EP 4 733 721 A1**

(12) **EUROPEAN PATENT APPLICATION**

(43) Date of publication:
**29.04.2026 Bulletin 2026/18**

(51) International Patent Classification (IPC):
***G01F 1/74*** *(2006.01)*    ***G01N 25/00*** *(2006.01)*
***G01N 33/00*** *(2006.01)*

(21) Application number: **24208318.6**

(22) Date of filing: **23.10.2024**

(52) Cooperative Patent Classification (CPC):
**G01F 1/74;** G01N 33/005

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **ABB SCHWEIZ AG**
**5400 Baden (CH)**

(72) Inventors:
• **KRAMER, Axel**
**8057 Zürich (CH)**

• **CASTRO, Pedro**
**8953 Dietikon (CH)**
• **ACKERMANN, Patric**
**64347 Griesheim (DE)**
• **MENDOZA, Francisco**
**69514 Laudenbach (DE)**

(74) Representative: **Maiwald GmbH**
**Engineering**
**Elisenhof**
**Elisenstrasse 3**
**80335 München (DE)**

(54) **SYSTEM AND METHOD FOR DETERMINING A GAS FLOW IN A GAS DELIVERY ARRANGEMENT**

(57)    A system (100) for determining a gas flow in a gas delivery arrangement (200), wherein the system (100) comprises: a data processing device (1), a flow meter (3) adapted to detect a flow of a gas mixture (11) comprising a target gas compound (51) and at least one impurity gas compound (53, 55), wherein the flow meter (3) is adapted to provide gas mixture flow information (5) based on the detected flow to the data processing device (1), a first compound concentration analyzer (7) calibrated to at least one first impurity gas compound (53) and adapted to detect a concentration of the first impurity gas compound (53) in the gas mixture (11), wherein the first compound concentration analyzer (7) is adapted to provide a first impurity concentration information (9) based on the detected concentration of the first impurity gas compound (53) in the gas mixture (11) to the data processing device (1), wherein the data processing device (1) is adapted to determine a target gas compound flow based on the gas mixture flow information (5) received from the flow meter (3) and the first impurity concentration information (9) received from the first compound concentration analyzer (7).

Fig. 1

EP 4 733 721 A1

**Description**

1. FIELD OF THE INVENTION

The invention relates to a system and a method for determining a gas flow in a gas delivery arrangement, a computer program product, and a computer-readable medium.

2. BACKGROUND

In gas delivery arrangements including applications of gas custody transfer, such as for natural gas or hydrogen, gas chromatography (GC) is usually applied to determine the composition of the transferred gas. The problem of the GC measurement principle is often the low measurement update rate and thus the lack of the possibility of detecting changing concentrations. Changing impurity concentrations in the gas may lead to erroneous mass determination, resulting *inter alia* in deteriorated gas quality, and for instance inaccurate billing. Furthermore, for some gases, only vague assumptions on the actual impurities in the gas streams at specific measurement locations exist. Their concentration as well as the temporal variations are accordingly unknown. However, rapid changes in the impurity concentration may occur if the gas will be fed in by various producers at multiple different positions of a gas delivery network, and, e.g. in case of hydrogen, utilizing for instance different gas production technologies. Moreover, the gas may be fed into the network from different storage locations (e.g. salt caverns, gas field storage, liquified storage) each characterized by different impurity gas species and varying concentrations.

Thus, there is a need for an improved system and method for determining a gas flow in a gas delivery arrangement.

3. SUMMARY OF THE INVENTION

The object is achieved by the present invention according to the independent claims. Preferred embodiments of the invention are provided in the dependent claims, the description and the accompanying figures.

The invention relates to a system for determining a gas flow in a gas delivery arrangement, wherein the system comprises a data processing device, a flow meter adapted to detect a flow of a gas mixture comprising a target gas compound and at least one impurity gas compound, wherein the flow meter is adapted to provide gas mixture flow information based on the detected flow to the data processing device, a first compound concentration analyzer calibrated to at least one first impurity gas compound and adapted to detect a concentration of the first impurity gas compound in the gas mixture, wherein the first compound concentration analyzer is adapted to provide a first impurity concentration information based on the detected concentration of the first impurity gas compound in the gas mixture to the data processing device, wherein the data processing device is adapted to determine a target gas compound flow based on the gas mixture flow information received from the flow meter and the first impurity concentration information received from the first compound concentration analyzer.

Hence, due to the combined employment of flow meter and compound concentration analyzer, an improved system for determining a gas flow in a gas delivery arrangement could be provided. In particular, the system may allow for a quick sampling rate and high sensitivity in the detection of impurities contained in the gas mixture. Furthermore, the system according to the present invention may be generally less expensive and less complex compared to the prior art. Hence, also lower capital expenditures und operational expenditures may be achieved. Furthermore, impurities may be better controlled, thus enabling a safeguarded high gas quality delivery. Finally, due to the less complex arrangement, also service and maintenance of the presented system may be enhanced compared to prior art solutions.

The gas delivery arrangement may include any type of gas distributing device or piping network, wherein a gas, e.g. a pure gas or a gas mixture, may be transferred from a starting or feeding-in point, e.g. at a source, gas producer or vendor site of the gas distribution arrangement, to an end point, e.g. at a customer or consumer site receiving said gas. In a particular embodiment, the gas delivery arrangement may correspond to a custody transfer arrangement and in particular refers to a hydrogen (H2) custody transfer arrangement.

The target gas may be the gas that is to be mainly distributed, and for which for example payment may be made. In a particular embodiment, the target gas may be hydrogen. Thus, the gas delivery system may be preferably a hydrogen delivery system.

The data processing device may include any suitable digital data processing means and may comprise a corresponding processor and temporal and/or permanent data storage devices, such as random-access memory (RAM), read-only memory (ROM), or any other kind of volatile or non-volatile memory. Data transmission may be enabled between the respective elements of the system, the flow meter and/or the first compound concentration analyzer via respective data transmission means, such as wired or wireless data transmission means. Furthermore, the data processing device may be enabled to receive input data from a user and/or may be enabled to send data information to the user. Furthermore, the data processing device may be enabled to control at least partially any of the elements provided in the system.

The gas mixture may include one or more target gas compounds, preferably one target gas compound. The target gas compound may in particular comprise or consist of H2. Likewise, the gas mixture may include one or more impurity gas compounds. A "gas compound" as such may include any suitable at least partially gaseous substrate. The impurity gas compound may include one or more of a gas compound that is different to the target gas compound. In some examples, the impurity gas compound may include one or more of nitrogen (N2), oxygen (02), water (H2O), methane (CH4), argon (Ar), carbon dioxide (CO2), ammonia (NH3) and carbon monoxide (CO). However also different impurity gas compounds may be conceivable.

The flow meter may include any device that is capable of determining or detecting directly or indirectly a measure of a flow of a gas. That is, the flow meter may include one or more devices suitable for measuring an amount of flow through a certain area, volume or space defined at for instance a piping of the gas delivery arrangement. The flow meter may accordingly determine and provide respective gas mixture flow information including information about the flow of the gas mixture in at least a portion of the gas delivery arrangement. Further, the flow meter may also include further information related to the detected flow of the gas mixture that may support a further processing via the data processing device.

The first compound concentration analyzer may include any device capable of detecting a concentration of one or more impurity gas compounds in a gas mixture, comprising for instance the impurity gas compound and the target gas compound. The first compound concentration analyzer may in particular not include or consist of a GC or a similar slow gas measuring devices. In other words, the first compound concentration analyzer may be a non-GC analyzer.

Generally, the first component concentration analyzer may be adapted to determine any desirable impurity concentration. In one example, considering hydrogen gas as target gas compound, the system may allow to safeguard a desired mass flow accuracy ($\Delta \dot{m}_{H2}/\dot{m}_{H2}$), wherein the relative hydrogen mass flow error is for instance less than 0.5% or 1%. Respective impurity gas compounds may comprise a maximum allowable concentration given in ppm, as summarized below, wherein the upper limits of impurity concentration are indicated, above which the impurity should be determined to ensure a calculated relative hydrogen mass flow error less than 0.5% and 1%, respectively.

**Table 1** - **Impurity concentrations**

| | Desired mass flow accuracy, $\Delta \dot{m}_{H2}/\dot{m}_{H2}$ | |
|---|---|---|
| Impurity | 0.50% | 1.00% |
| | Max. allowable conc. [ppm] | Max. allowable conc. [ppm] |
| H2O | 556 | 1111 |
| O2 | 313 | 625 |
| N2 | 357 | 714 |
| CO2 | 227 | 455 |
| CO | 357 | 714 |
| CH4 | 625 | 1250 |
| Ar | 250 | 500 |

The first compound concentration analyzer may be accordingly calibrated to the first impurity gas compound, wherein a calibration may include that said first impurity gas compound could be suitably detected in the gas mixture, e.g. with a low error and a high sensitivity. The calibration may be done manually or at least partially automatically or fully automatically. A concentration of the first impurity gas compound in the gas mixture may be detected and the data processing device may be accordingly informed via suitable first impurity concentration information or data about the concentration detected by the first compound concentration analyzer. The first impurity concentration information may accordingly contain the detected concentration of the first impurity gas compound and may contain potentially further information related to the detected impurity gas compound that may support a further processing via the data processing device. The first impurity concentration information may accordingly include information about one or more detected concentrations of one or more impurity gas compounds.

The target gas compound flow may represent the flow of the target gas compound in at least a portion of the gas delivery arrangement. In other words, target gas compound flow may represent an amount, e.g. a mass flow or a volume flow, of an essentially pure target gas compound. For instance, the system may accordingly enable to determine a flow of pure hydrogen flowing through the gas delivery arrangement by measuring a flow of gas mixture in the gas delivery arrangement. Exemplarily, the target gas compound flow may be determined in terms of energy (e.g. in Joules or kWh) during a given time interval using a calorific value, a volume (e.g. in m$^3$) or norm volume (e.g. in Nm$^3$) and/or a mass (e.g. in kg) of the target gas compound transferred during a given time interval (e.g. in s). However, the target gas compound flow may of course also be determined by using any other suitable physical expression.

The system may also comprise means adapted to log or store a record of the determined target gas compound flow and to output or report the respective information about the determined target gas compound flow, the respective impurity information and/or the gas mixture composition, e.g. to a user. This may allow for a traceability of the parameters determined by the system. According to one aspect, this may enhance financial billing or taxation of the gas delivered. In one example, the system may include outputting the gas component composition to a user. Further, in one example, the system may include to output the gas flow of desired gas species, such as the target gas compound, wherein the respective information may be for instance provided in kg/h or $Nm^2/h$. Further, in one example, the system may include logging the gas flow and/or an integral gas volume, wherein the respective information may be for instance logged in kg or $Nm^3$. Further, in one example, the system may include logging of the gas composition of the gas mixture. The logging, outputting or reporting may be performed based on user request or automatically at a regular interval, e.g. at predetermined time intervals.

In a preferred embodiment, the first compound concentration analyzer is adapted to detect the concentration of the first impurity gas compound in the gas mixture at a sampling rate of less than 60 seconds, more preferably less than 30 seconds, even more preferably less than 10 seconds, and most preferably less than 1 second.

Thus, a quick and reliable updating of the impurity concentration of the gas mixture may be achieved. Consequently, fluctuations of impurity concentrations may be determined at a fast in sampling rate, e.g. in real time, thus safeguarding a correct hydrogen billing and confidence of vendor and buyer of the gas mixture, in particular in custody transfer scenarios. As an additional value, the real-time determination of the target gas stream composition, e.g. a hydrogen gas stream, may be employed for improvement of process control in a hydrogen production unit or as a decision value for further usage. Further, the sampling may be combined with alarm triggering, e.g. if impurity concentrations exceed a certain threshold value.

In a preferred embodiment, the flow meter is a mass flow meter (MFM) adapted to detect a mass flow of the gas mixture, wherein the mass flow meter is adapted to provide gas mixture mass flow information based on the detected mass flow to the data processing device, and wherein the data processing device is adapted to determine a target gas compound flow based on the gas mixture mass flow information received from the mass flow meter and the first impurity concentration information received from the first compound concentration analyzer.

Thus, the flow of the gas mixture may be quickly and reliably determined based on the transferred mass of the gas mixture. The gas mixture mass flow information may contain information about the mass flow of the gas mixture through one or more parts or regions of the gas delivery arrangement. The mass flow information may accordingly include information about the mass flow, e.g. a mass of the gas mixture that has flowed through an area or space in a certain period of time. Accordingly, the flow of the target gas compound may be determined based on the detected gas mixture mass flow.

The flow meter may accordingly determine and provide respective gas mixture flow information including information about a mass flow of the gas mixture in at least a portion of the gas delivery arrangement and also further information related to the detected flow of the gas mixture that may support a further processing, e.g. via the data processing device. For instance, the target gas compound flow may be determined in terms of mass of the target gas compound transferred during a given time interval.

In a preferred embodiment, the flow meter is a volume flow meter (VFM) adapted to detect a volume flow of the gas mixture, wherein the volume flow meter is adapted to provide gas mixture volume flow information based on the detected volume flow to the data processing device, and wherein the data processing device is adapted to determine a target gas compound flow further based on the gas mixture volume flow information received from the volume flow meter and the first impurity concentration information received from the first compound concentration analyzer.

Thus, the flow of the gas mixture may be quick and reliably determined based on the transferred volume of the gas mixture. The gas mixture mass flow information may contain information about the volume flow of the gas mixture through one or more parts or regions of the gas delivery arrangement. The volume flow information may accordingly include information about the volume flow, e.g. a volume of the gas mixture that has flowed through an area or space in a certain period of time. Accordingly, the flow of the target gas compound may be determined based on the detected gas mixture volume flow.

The flow meter may accordingly determine and provide respective gas mixture flow information including information about a volume flow of the gas mixture in at least a portion of the gas delivery arrangement and also further information related to the detected flow of the gas mixture that may support a further processing, e.g. via the data processing device. For instance, the target gas compound flow may be determined in terms of volume of target gas compound transferred during a given time interval.

In a preferred embodiment, the data processing device is adapted to determine a calorific value of the gas mixture based on the first impurity concentration information.

Hence, the heating value or calorific value of the gas mixture may be reliably determined. Herein, known or measured heating values, which may also be referred to as calorific values, of the one or more target gas compounds may be used together with the mass flow and/or or volume flow of the gas mixture and/or the determined target gas compound flow to calculate an energetic value of the gas mixture flowing through the gas delivery arrangement, for instance in a certain time period.

Hence, a quality of the gas mixture with respect to its heating capabilities may be determined and for instance a respective billing of the gas mixture may be performed based on said calorific value. In some embodiments, additionally or alternatively to the first impurity concentration information, also a second impurity concentration information obtained from a second compound concentration analyzer, as described in detail further below, may be used to determine the calorific value of the gas mixture. For instance, the target gas compound flow may be determined in terms of energy during a given time interval using the calorific value.

In some examples, a precision in the determination of the heating value may be increased by additionally considering pressure and/or temperature information of the gas mixture. Hence, in some examples, the heating value may be either determined from the gas composition, and preferably by considering also the pressure and/or the temperature, or may in some examples be directly measured by an analyzer. This value may then be multiplied with the gas amount, e.g. provided in kg or moles, to determine the amount of energy supplied, which may then be for instance billed.

In a preferred embodiment, the system further comprises at least one pressure sensor, which is adapted to determine at least one pressure of the gas mixture, wherein the pressure sensor is adapted to provide pressure information based on the detected pressure to the data processing device, wherein the data processing device is adapted to determine the target gas compound flow further based on the received pressure information, and/or wherein the system further comprises at least one temperature sensor, which is adapted to determine at least one temperature of the gas mixture, wherein the temperature sensor is adapted to provide temperature information based on the detected temperature to the data processing device, wherein the data processing device is adapted to determine the target gas compound flow further based on the received temperature information.

Accordingly, the accuracy of the gas flow determination may be further enhanced based on additional pressure and/or temperature information of the gas mixture. Thus, reliability and precision in the determination of the target gas compound flow may be improved. The pressure sensor may include any suitable device adapted to directly or indirectly detect or determine a gas pressure. It will be appreciated that also more than one pressure sensor could be provided. The pressure may be measured or monitored in intervals or continuously or may be determined based on user request.

Also, the heating value of the gas mixture may be further refined based on the additional pressure information e.g. by taking into account minute changes of the heating value due to pressure variation.

Likewise, the temperature sensor may include any suitable device that is adapted to detect or determine a gas temperature. It will be appreciated that also more than one temperature sensor could be provided. The temperature may be measured or monitored in intervals or continuously or may be determined based on user request.

In some examples, when a flow meter may not directly measure a mass flow, the temperature information and/or pressure information may be used together with the gas composition information to calculate the net quantity of the gas species, which may then be accordingly billed and reported. This may for example include the calculation of the gas compressibility and/or the gas density, e.g. by an equation of state.

Also, the heating value of the gas mixture may be further refined based on the additional temperature information e.g. by taking into account minute changes of the heating value due to temperature variation.

In one example, the data processing device may be additionally or alternatively adapted to determine the calorific value of the gas mixture based on the received pressure information, and/or the data processing device may be additionally or alternatively adapted to determine the calorific value of the gas mixture based on the received temperature information.

The system, and particularly the data processing device, may also be adapted to log and store the determined temperature and/or pressure values of the gas mixture. The logging, outputting or reporting of said temperature and/or pressure values may be performed based on user request or automatically at a regular interval, e.g. at predetermined time intervals.

In a preferred embodiment, the system further comprises: a second compound concentration analyzer calibrated to at least one second impurity gas compound and adapted to detect a concentration of the second impurity gas compound in the gas mixture, wherein the second compound concentration analyzer is adapted to provide a second impurity concentration information based on the detected concentration of the second impurity gas compound in the gas mixture to the data processing device, wherein the data processing device is adapted to determine the target gas compound flow further based on the second impurity concentration information received from the second compound concentration analyzer.

Thus, a particular reliable determination of the target gas compound flow may be achieved. Accordingly, an increased accuracy in the determination of the target gas compound, e.g. H2, may be obtained. The second component concentration analyzer may include a paramagnetic oxygen analyzer and/or a laser-based analyzer, which may enable for specific gas analysis, fast response time and low maintenance operation. Generally, the type of the second compound concentration analyzer may be suitably chosen to collaborate and synergize with the selected type of the first compound analyzer and/or the selected type of the flowmeter. The second impurity concentration information may generally include information about one or more detected concentrations of one or more impurity gas compounds. In particular, if more than one impurity gas compound should be detected, a respective second compound concentration analyzer may be accordingly employed to facilitate the detection of the second impurity gas compound. Furthermore, also more than one second gas impurity compound may be detected simultaneously by the second compound concentration analyzer, for instance if a laser spectroscopic analyzer (LSA) is employed as second compound concentration analyzer.

Generally, the second impurity gas compound may be different compared to the first impurity gas compound. Nevertheless, the first impurity gas compound may also correspond to the second impurity gas compound. Hence, any description stipulated herein with respect to the first impurity gas compound and its determination accordingly applies to the second impurity gas compound.

In one example, the data processing device may be additionally or alternatively adapted to determine the calorific value of the gas mixture based on the second impurity concentration information received from the second compound concentration analyzer.

In a preferred embodiment, the second compound concentration analyzer is adapted to detect the concentration of the second impurity gas compound in the gas mixture at a sampling rate of less than 60 seconds, more preferably less than 30 seconds, even more preferably less than 10 seconds, and most preferably less than 1 second.

Thus, a quick and reliable updating of the impurity concentration of the gas mixture may be achieved. Consequently, fluctuations of impurity concentrations may be determined at a fast in sampling rate, e.g. in real time, thus safeguarding a correct hydrogen billing and confidence of vendor and buyer of the gas mixture, in particular in custody transfer scenarios. As an additional value, the real-time determination of the target gas stream composition, e.g. a hydrogen gas stream, may be employed for improvement of process control in a hydrogen production unit or as a decision value for further usage. Further, the sampling may be combined with alarm triggering, e.g. if impurity concentrations exceed a certain threshold value.

In a preferred embodiment, the first compound concentration analyzer and/or the second compound concentration analyzer includes one or more of an optical absorption analyzer, a thermal conductivity analyzer (TCA) or a speed of sound gas analyzer (SOS).

Thus, specific analyzers particularly selected for reliably determining the at least one second impurity gas compound may be employed either individually or in combination. The respective analyzers may be suitably adapted to determine one or more impurities in the gas mixture. The type of analyzer may be adapted to the respective gas mixture to be analyzed. An optical absorption analyzer may include or consist of, for instance, one or more spectroscopic analyzers operating based on the absorption of electromagnetic radiation in the ultraviolet, the visible and/or the infrared spectral regime. In one example, in case $H_2$ is employed as target gas compound, the first compound analyzer may be a TCA. The TCA may provide the advantage that it may be very sensitive to impurity admixtures to $H_2$, wherein the thermal conductivity of the impurity gas maybe different from the one of hydrogen. This difference may be well given for most of the impurity gas species occurring in industrial hydrogen processes such as $N_2$, $O_2$, $H_2O$, $CH_4$, Ar, $CO_2$, $NH_3$ and CO. For instance, the second component concentration analyzer may include or consist of an LSA. However, the present invention is not delimited thereto and may also include any other suitable compound concentration analyzer such as analyzers based on Raman-spectroscopy and so forth.

In a non-limiting example of the system, the first compound analyzer may be a TCA, the flow meter may be an MFM, and no second compound analyzer may be provided. In another non-limiting example of the system, the first compound analyzer may be a TCA, the second compound analyzer may be an LSA, and the flow meter may be an MFM. In another non-limiting example of the system, the first compound analyzer may be a SOS, the flow meter may be an MFM, and no second compound analyzer may be provided. In another non-limiting example of the system, the first compound analyzer may be a TCA, the flow meter may be a VFM, and no second compound analyzer may be provided. In another non-limiting example of the system, the first compound analyzer may be a SOS, the flow meter may be a VFM, and no second compound analyzer may be provided. In another non-limiting example of the system, the first compound analyzer may be a SOS, the second compound analyzer may be a TCA, and the flow meter may be an MFM. In another non-limiting example of the system, the first compound analyzer may be an LSA, the flow meter may be a MFM, and no second compound analyzer may be provided.

In a preferred embodiment, the second compound concentration analyzer is adapted to provide the second impurity concentration information to the first compound concentration analyzer, wherein the first compound concentration analyzer is adapted to use further calibration data based on the second impurity concentration information received from the second compound concentration analyzer, wherein the first compound concentration analyzer is adapted to provide a corrected first impurity concentration information based on the first impurity concentration and the second impurity concentration information to the data processing device, wherein the data processing device is adapted to determine a target gas compound flow based on the gas mixture flow information received from the flow meter and the corrected first impurity concentration information received from the first compound concentration analyzer.

Accordingly, the determination of the target gas compound flow may be further enhanced. In particular, precision in the determination of the first impurity concentration may be improved due to the corrected first impurity concentration information. Hence, an error in the determination of the target gas component flow may be reduced.

The first compound concentration analyzer and the second compound concentration analyzer may be suitably coupled to allow data-transfer between the respective analyzers, e.g. in a wired or wireless manner. In some examples, the system may be configured such that the data processing device receives and uses both, the corrected first impurity concentration information and the second impurity concentration information for the determination of the target gas compound flow. The second compound concentration analyzer may accordingly report a concentration of a second impurity compound to the data processing device and may, at the same time, report the concentration of the second impurity component to the first compound concentration analyzer to calculate a calibration function, which may enhance the determination of the first impurity compound by the first compound concentration analyzer. The further calibration data may include an employment of an adapted characteristic calibration map or an adapted set of calibration data.

Additional information of the flow meter, e.g. a mass flow meter or a volume flowmeter, may be provided to the data processing device and may be accordingly considered by the data processing device in the determination of the target gas component flow.

In one example, the data processing device may be additionally or alternatively adapted to determine a calorific value of the gas mixture based on the gas mixture flow information received from the flow meter and the corrected first impurity concentration information received from the first compound concentration analyzer.

The invention further relates to a method for determining a gas flow in a gas delivery arrangement, wherein the method comprises the steps of: detecting, by a flow meter, a flow of a gas mixture comprising a target gas compound and at least one impurity gas compound, providing gas mixture flow information based on the detected flow to a data processing device, calibrating a first compound concentration analyzer to at least one first impurity gas compound, detecting, by the first compound concentration analyzer, a concentration of the first impurity gas compound in the gas mixture, providing, by the first compound concentration analyzer, a first impurity concentration information based on the detected concentration of the first impurity gas compound in the gas mixture to the data processing device, determining, by the data processing device, a target gas compound flow based on the gas mixture flow information received from the flow meter and the first impurity concentration information received from the first compound concentration analyzer.

Hence, due to the combined employment of flow meter and compound concentration analyzer, an improved method for determining a gas flow in a gas delivery arrangement could be provided. In particular, the method may allow for a quick sampling rate and high sensitivity in the detection of impurities contained in the gas mixture. Furthermore, the method may allow for a generally less expensive and less complex determination of a target component flow compared to the prior art approaches. Hence, also lower capital expenditures und operational expenditures may be achieved. Furthermore, impurities could be better controlled enabling a safeguarded high quality gas delivery. Finally, the method may allow for a less complex detection arrangement, such that also service and maintenance of the presented system may be enhanced.

Any of the explanations stipulated above with respect to features of the system for determining a gas flow in a gas delivery arrangement according to the present invention accordingly apply to respective features of the method for determining a gas flow in a gas delivery arrangement according to the present invention.

Furthermore, an additional step of determining a calorific value of the gas mixture based on the first impurity concentration information and/or the second impurity concentration, preferably considering additional temperature information and/or pressure information, may be provided.

In a preferred embodiment, the method further comprises the steps of calibrating a second compound concentration analyzer to at least one second impurity gas compound, detecting a concentration of the second impurity gas compound in the gas mixture, providing, by the second compound concentration analyzer, a second impurity concentration information based on the detected concentration of the second impurity gas compound in the gas mixture to the data processing device, determining, by the data processing device, the target gas compound flow further based on the second impurity concentration information received from the second compound concentration analyzer.

Thus, a particular reliable determination of the target gas compound flow may be achieved. Further, an increased accuracy in the determination of the target gas compound, e.g. H2, may be achieved. The second component concentration analyzer may include a paramagnetic oxygen analyzer and/or a laser-based analyzer, which may enable for specific gas analysis, fast response time and low maintenance operation. Generally, the type of the second compound concentration analyzer may be suitably chosen to collaborate and synergize with the selected type of the first compound analyzer and/or the selected type of the flowmeter.

In particular, if more than one impurity gas compound should be detected, a respective second compound concentration analyzer may be accordingly employed to facilitate the detection of the second impurity gas compound.

In a preferred embodiment, the method further comprises the steps of providing, by the second compound concentration analyzer, the second impurity concentration information to the first compound concentration analyzer, using further calibration data for the first compound concentration analyzer based on the second impurity concentration information received from the second compound concentration analyzer, providing, by the first compound concentration analyzer, a corrected first impurity concentration information based on the first impurity concentration and the second impurity concentration information to the data processing device, and determining, by the data processing device, a target gas compound flow based on the gas mixture flow information received from the flow meter and the corrected first impurity concentration information received from the first compound concentration analyzer.

Accordingly, the determination of the target gas compound flow may be further enhanced. In particular, the precision in the determination of the first impurity concentration may be improved due to the corrected first impurity concentration information. Hence, an error in the determination of the target component flow may be reduced.

The first compound concentration analyzer and the second compound concentration analyzer may be suitably coupled to allow data-transfer between the respective analyzers. In some examples, the system may be configured such that the data processing device receives and uses both, the corrected first impurity concentration information and the second impurity concentration information for the determination of the target gas compound flow. The second compound concentration analyzer may accordingly report a concentration of a second impurity compound to the data processing device and may at the same time report the concentration of the second impurity component to the first compound concentration analyzer to calculate a calibration function, which may enhance the determination of the first impurity compound by the first compound concentration analyzer. The further calibration data may include the employment of an adapted characteristic calibration map or an adapted set of calibration data.

Additional information of the flowmeter, e.g. a mass flow meter or a volume flowmeter, may be provided to the data processing device and accordingly considered by the data processing device in the determination of the target gas component flow.

The invention further relates to a computer-program product comprising instructions, which, when executed by a data processing device, cause the data processing device to carry out and/or control at least partly the method according to the present invention.

The features and advantages outlined above in the context of the system and method for determining a gas flow in a gas delivery arrangement similarly apply to the computer program product described herein.

The features of the method according to the present invention may be implemented by respective suitable digital or computational means, which can include, for instance, one or more computers, apps and/or networks. The method may be at least partly computer-implemented, and may be implemented in software or in hardware, or in software and hardware. The data processing device may be any suitable computing device or means, such as a computer, an electronic control module etc., which may also be a distributed computer system. The data processing device may comprise one or more of a processor, a memory, a data interface, or the like.

The computer-program product may be stored/distributed on a suitable medium such as an optical storage medium or a solid-state medium, supplied together with or as part of other hardware, but may also be distributed in other forms, such as via the Internet or other wired or wireless telecommunication systems.

The invention further relates to a computer-readable medium comprising instructions which, when executed by a data processing device, cause the data processing device to carry out and/or control at least partly the method according to the present invention.

The features and advantages outlined above in the context of the system and method for determining a gas flow in a gas delivery arrangement and the computer-program product similarly apply to the computer-readable medium described herein.

Further features, examples, and advantages will become apparent from the following detailed description of preferred embodiments and the accompanying figures.


4. BRIEF DESCRIPTION OF THE DRAWINGS

For a better understanding of the present invention, and to illustrate its practicality, figures are provided in the following and reference is made thereto. It should be understood that the figures represent only exemplary embodiments and thus in no way limit the scope of the claimed invention. Identical or like-acting elements are indicated throughout by the same reference signs. Any reference signs in the claims should not be construed as limiting the scope of the claims. The figures are merely schematic representations and serve only to illustrate examples of the disclosure.

In the accompanying drawings,

Figure 1      is a schematical illustration of a system for determining a gas flow in a gas delivery arrangement according to an embodiment of the present invention,

Figure 2      is a schematical illustration of a system for determining a gas flow in a gas delivery arrangement according to an embodiment of the present invention,

Figures 3A to 3C      are flowcharts each illustrating a method for determining a gas flow in a gas delivery arrangement according to an embodiment of the present invention,

Figures 4A and 4B    are charts showing data sets of impurity concentrations of a gas mixture,

Figures 5A and 5B    are charts showing a relative error of mass flow of a target gas compound and an error reduction factor, respectively,

Figures 6A and 6B    are charts showing signals of a first compound concentration analyzer and a relative mass flow error, respectively,

Figure 7    is a chart showing a calibration function for a speed of sound of a gas mixture.

5. DESCRIPTION OF EMBODIMENTS

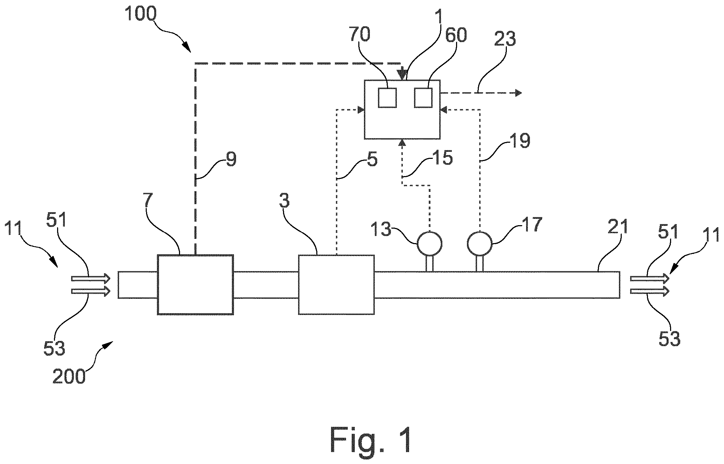

Figure 1 is a schematical illustration of a system 100 for determining a gas flow in a gas delivery arrangement 200 according to an embodiment of the present invention.

The gas delivery arrangement 200 comprises a pipe 21 through which a gas mixture 11 flows, wherein the gas mixture 11 comprises a target gas compound 51 and a first impurity gas compound 53, wherein the flow is indicated by respective arrows. As will be appreciated, the gas delivery arrangement 200 may of course also comprise a plurality of pipes 21. It will be appreciated, that the gas mixture 11 may also comprise more than one first impurity gas compound 53. The target gas component may be for instance H2 and the first impurity gas compound may be for instance N2.

The system 100 comprises a data processing device 1 adapted to receive information of different measurement devices, such as a first compound concentration analyzer 7 and a flow meter 3. The flow meter 3 is adapted to detect a flow of the gas mixture 11 comprising the target gas compound 51 and the impurity gas compound 53. The flow meter 3 is accordingly adapted to provide gas mixture flow information 5 based on the detected flow to the data processing device 1. For instance, the flowmeter 3 may be a mass flow meter adapted to detect a mass flow of the gas mixture 11, wherein the mass flow meter 3 is adapted to provide gas mixture mass flow information based on the detected mass flow to the data processing device 1. In another example, the flowmeter 3 may be a volume flow meter adapted to detect a volume flow of the gas mixture 11, wherein the volume flow meter is adapted to provide gas mixture volume flow information based on the detected volume flow to the data processing device 1.

The first compound concentration analyzer 7 is calibrated to the impurity gas compound 53 and adapted to detect a concentration of the first impurity gas compound 53 in the gas mixture 11. However, in case the gas mixture 11 comprises more than one and different impurities than the impurity gas compound 53, the first component concentration analyzer 7 may be accordingly calibrated in addition or alternatively to the other impurity gas compound(s) and may be accordingly adapted to detect also the respective concentration(s) of the other impurity gas compound(s) in the gas mixture 11. The first compound concentration analyzer 7 is adapted to provide a first impurity concentration information 9 based on the detected concentration of the first impurity gas compound 53 or, in case more than one impurity gas compound is present, of the detected concentrations of the more than one impurities in the gas mixture 11 to the data processing device 1. Thus, respective data may be transferred from the first compound concentration analyzer 7 to the data processing device 1, e.g. via wired or wireless data transfer means.

The data processing device 1 is adapted to determine a target gas compound flow based on the gas mixture flow information 5 received from the flow meter 3 and the first impurity concentration information 9 received from the first compound concentration analyzer 7. Hence, according to the above noted example, a flow of H2 could be determined. Accordingly, respective target gas compound flow information 23 could be provided, for instance presented to a user.

In case the flowmeter 3 is a mass flowmeter, the data processing device 1 is adapted to determine a target gas compound flow based on the gas mixture mass flow information 5 received from the mass flow meter and the first impurity concentration information 9 received from the first compound concentration analyzer 7. Likewise, if the flowmeter 3 is a volume flow meter, the data processing device 1 is adapted to determine a target gas compound flow based on the gas mixture volume flow information received from the volume flow meter and the first impurity concentration information 9 received from the first compound concentration analyzer 7.

The data processing device 1 could also be adapted to determine a heating value (calorific value) of the gas mixture based on the first impurity concentration information 9 and/or second impurity concentration 27. Herein, known or measured heating values, which may also be referred to as calorific values, of the one or more target gas compounds 51 may be used together with the mass flow and/or or volume flow of the gas mixture and/or the determined target gas compound flow to calculate an energetic value of the gas mixture 11 flowing through the gas delivery arrangement 200, for instance in a given time period. Furthermore, a precision in the determination of the heating value may be increased by additionally considering pressure information 15 and/or temperature information 19 of the gas mixture 11, which could be determined as described further below.

The first compound concentration analyzer 7 is adapted to detect the concentration of the first impurity gas compound 53 in the gas mixture 11 at a sampling rate of less than 60 seconds. However, in other examples, further reduced sampling times are possible, such as less than 30 seconds, less than 10 seconds or less than 1 second.

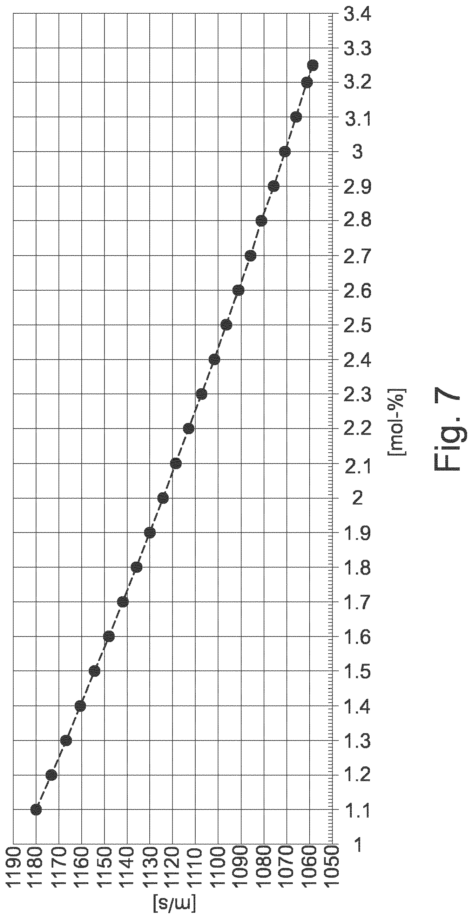

In the depicted embodiment, the first compound concentration analyzer 7 is a thermal conductivity analyzer. In case of a thermal conductivity analyzer, the reported impurity concentration may be unspecific. Hence, it may correspond to a signal that is dependent on all impurity concentrations contained multiplied by a concentration calibration factor. However, in other configurations an optical absorption analyzer or a speed of sound gas analyzer may be provided. In one example, alternatively to a separate speed of sound analyzer, the speed of sound value provided from an ultrasonic volume flow meter 3 calibrated for speed of sound measurement may also serve as a first impurity concentration information 9. The determination of the actual impurity concentration 10 may then be done internally by the data processing device 1 via a lookup table (cf. for instance Fig. 7) derived from the equation of state of the gas mixture 11.

In the depicted embodiment, the system 100 further comprises a pressure sensor 13, which is adapted to determine at least one pressure of the gas mixture 11. It will be appreciated that also more than one pressure sensor 13 could be provided in different configurations. The pressure sensor 13 is adapted to provide pressure information 15 based on the detected pressure to the data processing device 1 and the data processing device 1 is accordingly adapted to determine the target gas compound flow and heating value further based on the received pressure information 15.

In the depicted embodiment, the system 100 further comprises a temperature sensor 17, which is adapted to determine at least one temperature of the gas mixture 11. It will be appreciated that also more than one temperature sensor 17 could be provided in different configurations. The temperature sensor 17 is adapted to provide temperature information 19 based on the detected temperature to the data processing device 1, and the data processing device 1 is accordingly adapted to determine the target gas compound flow and heating value further based on the received temperature information 19.

Generally, the system 100 may comprise one or more pressure sensors 13 and/or one or more temperature sensors 17, as desired.

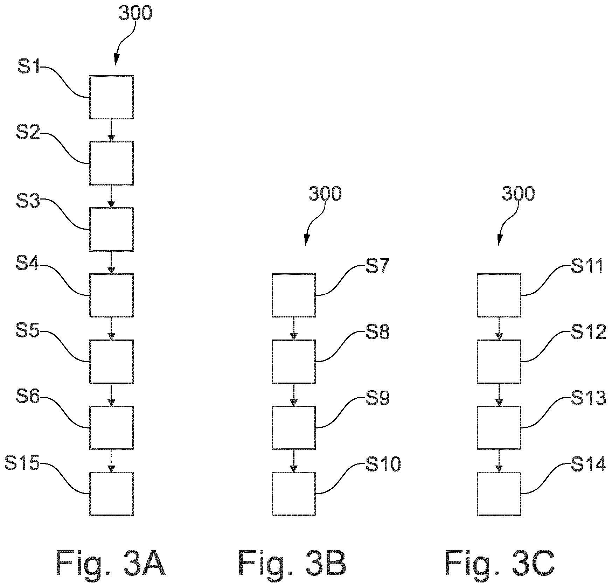

Further, a computer-program product 60 and a computer-readable medium 70 are shown, each comprising instructions, which, when executed by the data processing device 1, cause the data processing device 1 to carry out and/or control at least partly the method of any embodiments of the present invention, in particular the method 100 as illustrated in Figures 3A to 3C.

In the depicted embodiment, the computer-program product 60 and the computer-readable medium 70 are depicted as internal elements of the data processing device 1. However, it will be understood that in different embodiments, the computer-program product 60 and the computer-readable medium 70 may be provided remotely and operatively coupled to the data processing device 1, e.g. via respective means for wired or wireless data transfer.

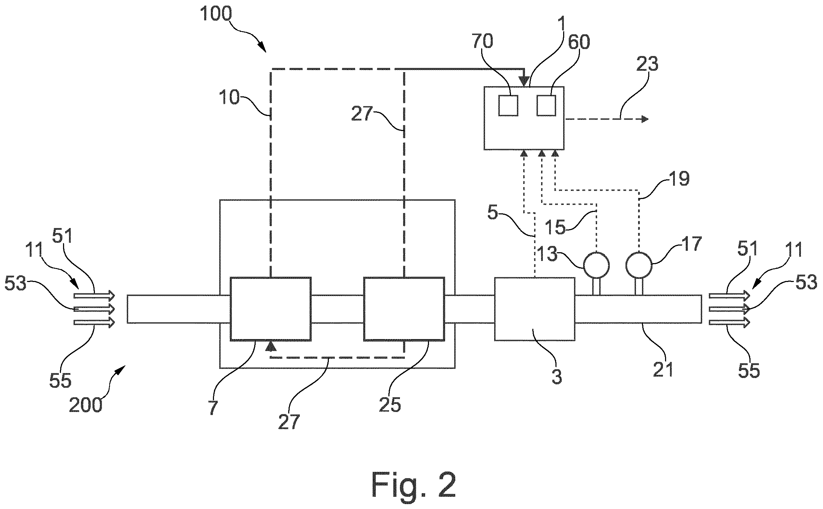

Figure 2 is a schematical illustration of a system 100 for determining a gas flow in a gas delivery arrangement 200 according to another embodiment of the present invention. As will be appreciated, similar elements of this embodiment as were already described with respect to the previous embodiment shown in figure 1 are not reiterated herein for the sake of brevity.

In the embodiment of the system 100 depicted in figure 2, a second impurity gas compound 55 is additionally present in the gas mixture 11. It will be appreciated, that the gas mixture 11 may also comprise more than one second one impurity gas compound 53. The target gas component may be for instance H2, the first impurity gas compound may be for instance N2 and the second impurity gas compound may be for instance CH4.

In the depicted embodiment, the system 100 further comprises a second compound concentration analyzer 25 calibrated to at least one second impurity gas compound 55 and adapted to detect a concentration of the second impurity gas compound 55 in the gas mixture 11. The second compound concentration analyzer 25 is adapted to provide a second impurity concentration information 27 based on the detected concentration of the second impurity gas compound 55 in the gas mixture 11 to the data processing device 1.

The data processing device 1 is accordingly adapted to determine the target gas compound flow further based on the second impurity concentration information 27 received from the second compound concentration analyzer 25.

The second compound concentration analyzer 25 is adapted to detect the concentration of the second impurity gas compound 55 in the gas mixture 11 at a sampling rate of less than 60 seconds. However, in other examples, further reduced sampling times are possible, such as less than 30 seconds, less than 10 seconds or less than 1 second.

In the depicted embodiment, the second compound concentration analyzer 25 is a laser spectroscopic analyzer. However, in other configurations also other kinds of optical absorption analyzers, thermal conductivity analyzers speed of sound gas analyzers may be provided.

In the depicted embodiment, the second compound concentration analyzer 25 is further adapted to provide the second impurity concentration information 27 to the first compound concentration analyzer 7. The first compound concentration analyzer 7 is adapted to use further calibration data based on the second impurity concentration information 27 received from the second compound concentration analyzer 25 and to provide a corrected first impurity concentration information 10 based on the first impurity concentration and the second impurity concentration information 27 to the data processing device 1.

The data processing device 1 is adapted to then determine a target gas compound flow based on the gas mixture flow information 5 received from the flow meter 3 and the corrected first impurity concentration information 10 received from the first compound concentration analyzer 7.

Figures 3A, 3B and 3C depict flowcharts each illustrating a method 300 for determining a gas flow in a gas delivery arrangement 200 according to an embodiment of the present invention. Figures 3A, 3B and 3C refer also to elements of the system 100 above described in detail with respect to Figures 1 and 2, which are not reiterated herein for the sake of brevity.

As depicted in Figure 3A, the method 300 comprises the step S1 of detecting, by the flow meter 3, the flow of the gas mixture 11 comprising the target gas compound 51 and the at least one impurity gas compound 53, 55. The method 300 further comprises the step S2 of providing gas mixture flow information 5 based on the detected flow to a data processing device 1. The method 300 further comprises the step S3 of calibrating the first compound concentration analyzer 7 to the at least one first impurity gas compound 53. The method 300 further comprises the step S4 of detecting, by the first compound concentration analyzer 7, the concentration of the first impurity gas compound 53 in the gas mixture 11. The method 300 further comprises the step S5 of providing, by the first compound concentration analyzer 7, a first impurity concentration information based on the detected concentration of the first impurity gas compound 53 in the gas mixture 11 to the data processing device 1. The method 300 further comprises the step S6 of determining, by the data processing device 1, a target gas compound flow based on the gas mixture flow information 5 received from the flow meter 3 and the first impurity concentration information received from the first compound concentration analyzer 7. Optionally, the step S1 may further include the step of determining a mass flow and/or a volume flow of the gas mixture 11. Furthermore, an additional step S15 of determining a heating value (calorific value) of the gas mixture based on the first impurity concentration information 9 and/or the second impurity concentration 27, preferably considering also the temperature information 19 and/or the pressure information 15, may be provided.

As depicted in Figure 3B, the method 300 comprises the step S7 of calibrating the second compound concentration analyzer 25 to at least one second impurity gas compound 55. The method 300 further comprises the step S8 of detecting a concentration of the second impurity gas compound 55 in the gas mixture 11. The method 300 further comprises the step S9 of providing, by the second compound concentration analyzer 25, the second impurity concentration information 27 based on the detected concentration of the second impurity gas compound 55 in the gas mixture 11 to the data processing device 1. The method 300 further comprises the step S10 of determining, by the data processing device 1, the target gas compound flow further based on the second impurity concentration information 27 received from the second compound concentration analyzer 25.

As depicted in Figure 3C, the method 300 comprises the step S11 of providing, by the second compound concentration analyzer 25, the second impurity concentration information 27 to the first compound concentration analyzer 7. The method 300 further comprises the step S12 of using further calibration data for the first compound concentration analyzer 7 based on the second impurity concentration information 27 received from the second compound concentration analyzer 25. The method 300 further comprises the step S13 of providing, by the first compound concentration analyzer 7, a corrected first impurity concentration information 10 based on the first impurity concentration and the second impurity concentration information 27 to the data processing device 1. The method 300 further comprises the step S14 of determining, by the data processing device 1, a target gas compound flow based on the gas mixture flow information 5 received from the flow meter 3 and the corrected first impurity concentration information 10 received from the first compound concentration analyzer 7.

It will be understood that the method according to the present invention is not limited to the above noted order of method steps. Quite to the contrary, the method steps may be also provided in a different order and one or more of the above noted method steps may be removed, or further method steps may be added, as desired.

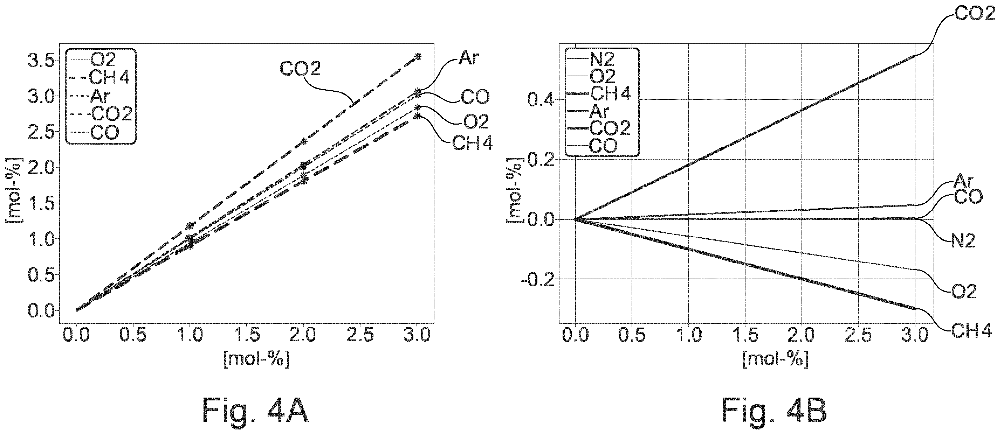

Figures 4A is a chart showing data sets of measured impurity concentrations of a gas mixture comprising H2 as a target gas compound and one of 02, CH4, Ar, CO2 or CO as impurity gas compound. In Figure 4A, on the x-axis the real impurity concentration given in mol-% is provided. On the y-axis the measured molar fraction or percentage given in mol-% of the respective impurity gas compound is provided. In the present example, the impurity concentration measurements are performed by a TCA. Hence, Figure 4A represents TCA-reported impurity concentrations $X'_1$ as a function of real impurity concentration $X_i$. In the depicted embodiment, experimental data sets of responses of the TCA are shown as crosses, wherein the TCA is calibrated for N2 contained in H2. Furthermore, linear fits represented as dashed lines were applied to the experimental data sets.

Figures 4B is a chart showing data sets of impurity concentrations of a gas mixture comprising H2 as a target gas compound and one of N2, 02, CH4, Ar, CO2 or CO as impurity gas compound. In Figure 4B, on the x-axis the real impurity concentration given in mol-% is provided. On the y-axis the impurity concentration error given in mol-% of the respective impurity gas compound is provided. Hence, Figure 4B shows the resulting absolute impurity concentration errors based on the TCA-measurements shown in Figure 4A.

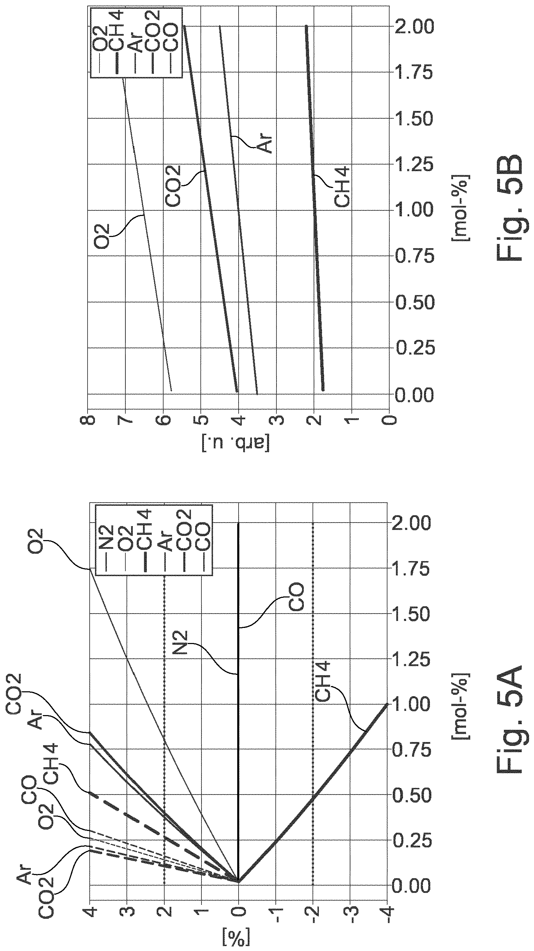

Figures 5A is a chart showing a relative error of mass flow of a target gas compound, the gas mixture comprising H2 as a target gas compound and one of N2, 02, CH4, Ar, CO2 or CO as impurity gas compound. In Figure 5A, on the x-axis the impurity concentration given in mol-% is provided. On the y-axis the relative mass flow error of the target gas, which is H2 in the depicted case, in molar fraction or percentage is provided. The solid lines depict the relative mass flow errors when a first compound concentration analyzer is provided, which is a TCA calibrated for N2 in H2 in the presented case, and when a flow meter is provided, which is a mass flow meter in the presented case. The dashed lines indicate the respective relative mass flow error without provision of a first compound concentration analyzer when respective impurity gas compounds are provided. In the depicted chart, the lines for CO and N2 correspond to each other.

Figures 5B is a chart a reduction factor of a relative mass flow error of the target gas compound, here H2, using the method of the invention compared to the case using only a mass flow meter and assuming that the gas mixture 11 is pure H2 plotted versus a respective impurity gas compound concentration given in molar fraction or percentage. For CO, the reduction factor approaches infinity, as the relative mass flow error vanishes (cf. also Fig. 5A).

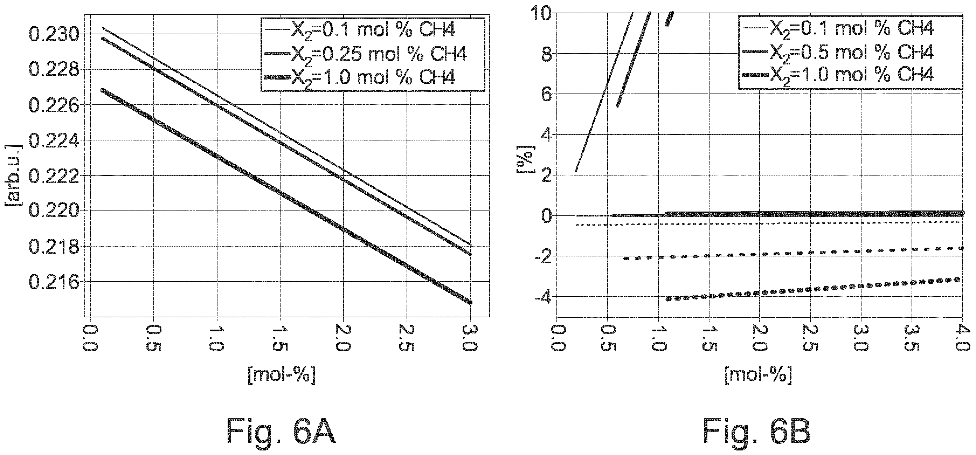

Figure 6A is a chart showing signals of a first compound concentration analyzer for different impurity concentrations of CH4. In Figure 6A, on the x-axis the primary impurity concentration ($X_1$) given in mol-% is provided. On the y-axis the dimensionless internal TCA signal is provided. Figure 6A accordingly shows the internal TCA signal $S(X_1,X_2)$ as a function of the molar concentration $X_1$ of N2 for varying molar fractions $X_2$ of CH4. The inverse function represents the calibration function $cal_R(S(X_1,X_2),X_2)$ used to calculate the output $X_R'$ of the TCA for different concentrations of the second impurity $X_2$, as will be explained further below.

Figure 6B is a chart showing a relative mass flow error for different impurity concentrations of N2 and CH4 ($X_1$ and $X_2$). In Figure 6B, on the x-axis the total impurity concentration ($X_1 + X_2$) given in mol-% is provided. On the y-axis, the relative mass flow error of the target gas compound (here H2) is provided. The error ($\Delta_r(\dot{m}_{H2}')$) is calculated as described further below. In the presented example, tertiary mixtures of H2 with N2 and CH4 are considered. The dashed lines show the relative mass flow error (calculated with equation 3a, using equation 1a and equations 2a and 2b) without compensation for the impurities

($X_1' = 0$) as described herein. Dotted lines show the resulting mass flow error using only a TCA calibrated for N2 (calculated with equation 3a, using equation 1a and equations 2a and 2b). Solid lines show the mass flow error using a parametric TCA calibration for N2 in H2 for varying concentrations ($X_2$) of CH4 (using equation 3a with equations 1a, 2a and 5). Further, in the presented example, the sake of simplicity, a perfect flow meter calibration is assumed.

Figure 7 is a chart showing a calibration function for a speed of sound of a gas mixture. In Figure 7, on the x-axis the impurity concentration of an impurity gas compound, here N2, given in mol-%, present in a target gas compound, here H2, is depicted. On the y-axis the speed of sound in the gas mixture given in meter per second is depicted. As is apparent, the speed of sound decreases with increasing impurity concentration.

In the following, several different exemplary embodiments and scenarios are discussed with respect to the above identified figures 4A, 4B, 5A, 5B, 6A, 6B and 7 to facilitate the understanding of the present invention. In the subsequent examples, H2 is considered as target gas compound, and different examples for gas compounds of first and second impurity gas compounds are considered. However, it is understood that the below examples are merely exemplarily and that also, for instance, a different target gas compound and different first and second impurity gas compounds may be considered. The respective exemplary equations may be accordingly adapted to the needs and configurations in accordance with the system and the method described above.

In a first embodiment, the system comprises a TCA as a first compound concentration analyzer and an MFM as flow meter. The target gas compound is assumed to be hydrogen. TCA and MFM report their data to the data processing device, which calculates the net mass flow quantity of hydrogen in real-time. The real mass-flow of hydrogen, which may be a billed quantity, $\dot{m}_{H2}$, can be calculated from the total mass flow measured by the MFM, $\dot{m}$, when the gas composition is known. In the case of a single impurity (e.g. N2), the mass-flow of hydrogen can be calculated by:

$$\dot{m}_{H2} = \dot{N} \cdot M_{H2} \cdot X_{H2} \quad (eq.\,1a),$$

wherein

$$\dot{N} = \frac{\dot{m}}{X_1 \cdot M_{N2} + (1 - X_1) \cdot M_{H2}} \quad (eq.\,1b)$$

In the above equations, $\dot{m}_{H2}$ is the real hydrogen mass flow, which also may be referred to as ground truth, $\dot{N}$ is the real molar flow of all gas species, $M_{H2}$ is the molar mass of hydrogen, $X_{H2}$ is the real molar fraction of hydrogen, $\dot{m}$ is the mass flow, measured by MFM, $X_1$ is the real molar fraction of the first impurity gas compound and $M_{N2}$ is the molar mass of nitrogen.

As will be appreciated, the presented concept is independent of an equation of state, pressure and temperature and only depends on the variables $X_1$ and $\dot{m}$. In the following, the measurement uncertainty of these quantities is elaborated. Error prone measured quantities or quantities calculated from those error prone measured quantities will be denoted with a prime (').

In an example of this embodiment, the TCA is calibrated for a binary mixture of N2 and H2, of which the molar fractions are denoted with $X_1'$ and ( $1 - X_1'$ ), respectively. In this calibration, a calibration function $cal_1$ is determined, such that an obtained signal $S(X_1)$ generated inside the analyzer is mapped to the true molar fraction $X_1$:

$$X_1' = cal_1(S(X_1)) = X_1 + \Delta X_1' ,$$

wherein $X_1'$ is the molar fraction or concentration of first impurity gas compound, $cal_1$ is the calibration function of TCA to map the signal $S(X_i)$ generated inside the TCA from a binary mixture of H2 with molar fraction $X_i$ of impurity gas i to the molar impurity fraction $X_1$ (i=1...n), and $\Delta X_1'$ is the error of the impurity concentration, reported by the gas analyzer.

This mapping is achieved e.g. by minimizing the sum of the squared deviation $(\Delta X_1')^2$ of measurement and fit data. The TCA reports the measured molar fraction of the impurity, $X'_1$ , to data processing device. In the presented example, the mapping is only perfect for the calibration gas (here: N2-H2 mixture). For binary mixtures of H2 with gas other than nitrogen, the obtained signal $S(X_i)$ will be different and hence a separate calibration function $cal_i$ would be necessary. The reported impurity concentration $X_1' = cal_1(S(X_i))$ is shown in Figure 4A for impurity gas species other than nitrogen, while the TCA is calibrated for N2 in H2 (i=2,...n). From this, the resulting absolute concentration error of the impurity $\Delta X_1' = (X_1' - X_i)$ can be calculated and is shown accordingly in Figure 4B.

The data processing device is adapted to calculate the hydrogen mass-flow, $\dot{m}'_{H2}$ , as indicated in the following equations, respectively:

$$\dot{m}'_{H2} = \dot{N}' \cdot M_{H2} \cdot X'_{H2} \quad (eq.\,2a),$$

wherein

$$\dot{N}' = \frac{m}{X_1' \cdot M_{N2} + (1 - X_1') \cdot M_{H2}} \quad (eq.\,2b)$$

In the above equations, $\dot{N}'$ is the calculated molar flow of all gas compounds, and $X_1'$ is the measured molar fraction of the first impurity gas compound reported by the first gas compound analyzer. Generally, the real mass flow of the respective impurity compound i (i=1... n) may be expressed as $\dot{m}_i$. The calculation of $\dot{N}'$ is based on the measured mass flow, $\dot{m}$ , reported by the MFM and on the TCA-reported impurity concentration $X_1'$ . The MFM-reported mass flow is assumed for simplification in this example without measurement error.

Figure 5A shows the relative error of the calculated hydrogen mass flow $\Delta_r(\dot{m}'_{H2})$ with respect to the real hydrogen mass flow, expressed by the following equation:

$$\Delta_r(\dot{m}'_{H2}) = (\dot{m}'_{H2} - \dot{m}_{H2})/\dot{m}_{H2} \quad (eq.\,3a),$$

wherein

$$\dot{m}_{H2} = \dot{N} \cdot M_{H2} \cdot X_{H2} \quad (eq.\,1a)$$

Scenario 1 - System without a gas composition analyzer and considering a single impurity *i*, (*i*=1):

In this scenario, the mass flow meter measures $\dot{m} = \dot{m}_{H2} + \dot{m}_1$. When knowledge about the gas composition is not available, the impurity concentration must be assumed to be zero, i.e., $X1 = 0$. Hence, the data processing device is adapted to report $\dot{m}'_{H2} = \dot{m} = \dot{m}_{H2} + \dot{m}_1$. In this case, the relative error is:

$$\Delta_r(\dot{m}'_{H2}) = \frac{\dot{m}'_{H2} - \dot{m}_{H2}}{\dot{m}_{H2}} = \frac{\dot{m}_1}{\dot{m}_{H2}} = \frac{X_1' \cdot M_1}{(1 - X_1') \cdot M_{H2}} \approx \frac{X_1' \cdot M_1}{M_{H2}} \;(eq.\,4),$$

wherein $M_1$ is the molar mass of the first impurity gas compound. The relative error, in approximation $X_1' \ll 1$ is directly proportional to the fraction of the molar mass of the impurity to the molar mass of hydrogen. Furthermore, the relative error is proportional to the real (unknown) impurity concentration. In this scenario, one would incur a relative error of hydrogen mass flow of several percent for impurity concentrations of 0.2-0.3 mol-%. These errors are illustrated by the colored dashed lines in Fig. 5A.

Scenario 2 - Analyzer calibrated for the only impurity with relevant concentration to affect calculated mass flow accuracy (e.g., N2 as single impurity and a TCA calibrated for N2 in H2):

In this scenario, the additional mass flow caused by the impurity is perfectly taken into account by the analyzer and data processing device and the calculated H2 mass flow $\dot{m}_{H2}'$ is equal to the real H2 mass flow $\dot{m}_{H2}$ resulting in zero relative error (cf. line of N2 in Fig. 5A, congruent with line of CO).

In a real measurement scenario, including also device errors, the accuracy of the flow meter and the accuracy and sensitivity of the analyzer generally define the system accuracy. Assuming a flow meter error <0.5% (of reading) and a TCA error of $\Delta X_i'$ = 300 ppm (1% full scale error at 3% impurity concentration), the resulting combined error of calculated mass flow is $\Delta_r \dot{m}_{H2}'$ < 1%.

Scenario 3 - Gas compound analyzer calibrated for N2 in H2 and an additional unknown impurity other than N2:

In this scenario, the presented solution may allow to detect the impurity with similar sensitivity compared to N2 (as shown in Fig. 4A). Using this information, the hydrogen mass flow can be calculated from the total mass flow reported by the MFM. Even in this case the solution can improve the hydrogen mass flow accuracy in reducing the relative error $\Delta_r$ $(\dot{m}_{H2}')$ by a factor of 2 up to 7.5 with respect to the case without an analyzer (cf. above case 1). The level of improvement may depend on the respective impurity gas species ($i$) (as shown in Figure 5B). The resulting relative mass flow error $\Delta_r$ $(\dot{m}_{H2}')$ generally stems from two sources:

a. The impurity concentration error $\Delta X_1' = X_1' - X_i$ (shown in Fig. 4B), wherein $X_i$ is the real molar fraction of the impurity gas compound $i$, ($i$=1... n). In the exemplary combination with a TCA, the TCA reports lower impurity concentrations ( $\Delta X_1' < 0$ ) for gases significantly lighter than N2 (e.g. CH4) and higher concentrations ( $\Delta X_1' > 0$ ) for gases significantly heavier than N2 (e.g. CO2) (cf. Figure 4B).

b. The molar mass of the impurity: As the gas species is not known, the molar mass of N2 is to be assumed for the calculation.

Favorably, the error (b) from assuming $M_{N2}$ is compensated partly by the error $\Delta X_1'$ (a), resulting in a low hydrogen mass flow error $\Delta_r(\dot{m}_{H2}')$ even with unknown impurity species. As a further advantage, the TCA can report the impurity concentration of unknown impurities also with a low error (cf. Fig. 4B) and can serve as an indicator for the overall impurity concentration to decide if further specific gas compound analyzers are advantageous at the measurement location.

Hence, the presented concept is also robust for unknown impurities other than nitrogen: E.g., for binary mixtures of hydrogen with other possible impurities in hydrogen streams, such as 02, CH4, Ar, CO2, and CO, as depicted in in Figure 4A. Considering for instance CO2 as an impurity, the relative error $\Delta_r(\dot{m}_{H2}')$ is below 2% if the CO2 concentration is below 0.39 mol% (cf. Fig. 5A). Considering for instance Ar as an impurity, the relative error $\Delta_r(\dot{m}_{H2}')$ is below 2% if the Ar concentration is below 0.36 mol% (cf. Fig. 5A). Considering for instance 02 as an impurity, the relative error $\Delta_r(\dot{m}_{H2}')$ is below 2% if the 02 concentration is below 0.78 mol-% (cf. Fig. 5A). Considering for instance CH4 as an impurity, the relative error $\Delta_r(\dot{m}_{H2}')$ is below 2% if the CH4 concentration is below 0.45 mol-% (cf. Fig. 5A). Considering, for instance, CO as an impurity, there is virtually no mass flow error within the full span of the analyzer.

In a second embodiment, for increased accuracy of hydrogen mass flow determination one or more second compound concentration analyzers may be added. Those may be, e.g. an optical absorption analyzer, a thermal conductivity analyzer or a speed of sound gas analyzer, a paramagnetic oxygen analyzer and/or a laser-based analyzer for specific gas analysis, fast response time and low maintenance operation. The additional gas analyzers report the mole fractions of the analyzed impurity gas species $X_i'$ to the data processing device ($i$=2... n). The data processing device then uses the $X_i'$ to compute the molar flow by using the following equation:

$$\dot{N}' = \frac{\dot{m}}{\sum_i^n (X_i' \cdot M_i) + X_R' \cdot M_{N_2} + \left(1 - \sum_i^n (X_i') - X_R'\right) \cdot M_{H_2}} \quad (eq.\,5)$$

whereas $X_R'$ is the molar fraction of N2, when an impurity gas compound other than N2 is present, i.e. the corrected molar fraction of N2 in the stream, $X_i'$ is the molar fraction of the impurity gas compound $i$, ($i$=2...n) other than the gas compound the non-gas specific analyzer (e.g. the first gas compound analyzer) is calibrated for (measured by specific analyzer or from measurement deduced), and $M_i$ is the molar mass of the impurity gas compound i. $X_R'$ could be calculated from the TCA-reported apparent N2 mole percentage $X_1'$ by an appropriate correction function based on the known sensitivity differences of the thermal conductivity analyzer to the other impurities as shown in Fig. 4A. The simplest correction formula would e.g. be to correct the apparent mole fraction $X_1'$ by linear compensation: $X_R' = X_1' - \sum cal_1(S(X_i'))$, whereas $cal_1(S(X_i'))$ is the reported impurity concentration by TCA for the molar fraction $X_i'$ of impurity $i$ as shown in Fig 4A. Generally, $cal_i$ could be considered as the calibration function of TCA to map the signal $S(X_i)$ to the molar impurity fraction $X_i$. Other correction functions may be potentially derived from kinetic gas theory.

One such implementation is presented as a specific example for the often-occurring situation, in which N2 and CH4 are the only impurities with molar fractions $X_1$, $X_2$ exceeding 300ppm. Accordingly, a tertiary mixture of H2 with N2 and CH4 may be considered, and, for this combination of impurities, e.g. an LSA may be combined with the TCA, which corresponds to a configuration as depicted in Figure 2. In the presented example, the LSA is adapted to measure the specific concentration $X'_2$ of CH4 and could accordingly report molar concentration $X'_2$ to the TCA. This quantity is then used to select the proper calibration function $cal_R(S(X_1,X_2),X_2)$. With this function, the TCA could calculate the corrected concentration $X_R'$ of the first impurity (here N2). The calibration function $cal_R(S(X_1,X_2),X_2)$ could be derived from the internal signal $S(X_1,X_2)$ e.g. by a multi-point calibration at different levels of $X_2$. In other words, $cal_R$ could be considered as the calibration function of the TCA to map the signal $S(X_1,X_2 ... X_i)$ to the molar impurity fraction $X_1$, considering the concentrations $X_2 ... X_i$ of additional impurities that can be reported by the gas-specific first and/or second compound concentration analyzers.

Figure 6A shows the respective TCA internal signal $S(X_1,X_2)$ for three different CH4 concentrations $X'_2$, namely for 0.1 mol-%, 0.25 mol-% and 1.0 mol-% CH4. In this respect, the signal generated inside the TCA from a tertiary mixture of H2 with molar fraction $X_1$ of a primary impurity and a with molar fraction $X_i$ of a secondary impurity $i$ may be referred to as $S(X_1,X_i)$.

The mass flow of hydrogen is calculated and reported by the data processing device (at least) from the quantities $X'_2$ and $X'_R$ reported by the LSA and the TCA as well as $\dot{m}$ reported by the MFM. The calculation is performed as described in equation 2a using $\dot{N}'$ from equation 5 as input. The resulting relative mass flow error for the combined solution is exemplarily plotted in Figure 6B. The error is close to zero for all considered CH4 and N2 concentrations. The relative error for using a TCA alone (dotted curves in Fig. 6B) is <4% for CH4 concentrations <1 mol-%. Without an analyzer, the relative error would even exceed 10% already at a combined impurity concentration $(X_1' + X'_2)$ <1% .

In a third embodiment, a speed-of-sound (SOS) gas analyzer and a mass-flow meter report data to the data processing device. The SOS analyzer may be on purpose chosen to be unspecific to the impurity species, but very sensitive to admixtures of any possible impurities.

The analyzer may be calibrated for a binary mixture of the most abundant impurity, e.g. Nitrogen (N2) in Hydrogen (H2) to report the molar fraction $X_1'$ of the impurity to the data processing device. The computation is analog to the first embodiment. The third embodiment has the advantage, that the speed of sound $c = \sqrt{\frac{\gamma R T}{M}}$ at constant temperature inside the analyzer is only a function of the adiabatic index or exponent $\gamma$, which may be also expressed as $\gamma = c_p/c_v$ and the molar mass. As $\gamma \approx 1.5$ for nearly all gas species, the dependency on the molar mass $M$ yields a strong sensitivity of the speed of sound to impurities in hydrogen. The functional dependence of the speed of sound in a gas mixture can be well described with the simple mixing rule, summing over all species $i$. The speed of sound of the binary gas mixture as $c_{mix}$ could be expressed as:

$$c_{mix} = \sqrt{\frac{\sum X_i C_{p_i}}{\sum X_i C_{v_i}} R T \cdot \left(\sum X_i M_i\right)^{-1}}$$

wherein $c_{p_i}$ is the specific heat capacity at constant pressure of impurity $i$, and $c_{v_i}$ is the specific heat capacity at constant volume of impurity $i$.

This ensures the same functional dependence for all possible impurities and enables aggregate detection of impurities of different species with a low error on the calculated H2 mass flow. The dependence $c_i \propto \sqrt{\dfrac{1}{M_i}}$ of the speed of sound $c_i$ of the gas species $i$ on molar mass here compensates partially for the different molar masses of unknown species. The following table shows the calculated hydrogen mass flow for a flow of 1 mol of gas per time unit:

**Table 2** - **Calculated hydrogen mass flow for a flow of 1 mol of gas per time unit**

| | | | | | | flow of 1mol/time-unit | | | | |
|---|---|---|---|---|---|---|---|---|---|---|
| | | | $c_i$ | $c_{mix}$ | $X'_1$ | $X'_1 \cdot M_{N2}$ / $M_{N2}$ | $X_i \cdot M_i$ | $\dot{m}_{H2}$ | | $\Delta_r(\dot{m}_{H2})$ |
| **impurity** | **M_i** | | **SOS pure** | **SOS mix** | measured | calculated | real | real | calculated | **Error in** |
| in mol % | molar mass | | in m/s (1atm, 0°C) | | N2 mol% | g/mol | g/mol | g/mol | g/m | **H2 mass-flow** |
| 2% H2O | 18 | g/mol | 387.1 | 1170.6 | 1.24% | 0.3472 | 0.36 | 1.96 | 1.973 | 0.67% |
| 2% O2 | 32 | g/mol | 314.8 | 1107 | 2.30% | 0.644 | 0.64 | 1.96 | 1.956 | -0.23% |
| 2% N2 | 28 | g/mol | 337 | 1124.3 | 2% | 0.56 | 0.56 | 1.96 | 1.960 | 0.00% |
| 2% CO2 | 44 | g/mol | 258.1 | 1058.4 | 3.25% | 0.91 | 0.88 | 1.96 | 1.932 | -1.45% |
| 2% CO | 28 | g/mol | 336.9 | 1124.3 | 2% | 0.56 | 0.56 | 1.96 | 1.960 | 0.00% |
| 2% CH4 | 16 | g/mol | 430.7 | 1180.5 | 1.10% | 0.308 | 0.32 | 1.96 | 1.973 | 0.65% |
| 2% Ar | 40 | g/mol | 307.9 | 1076.1 | 2.9% | 0.812 | 0.8 | 1.96 | 1.946 | -0.70% |

The above results only use one gas unspecific analyzer without considering the measurement accuracy of such. If the SOS analyzer is calibrated for H2-N2 mixtures and if N2 is the single impurity in the gas, the H2 mass flow is reported correctly. But also, for admixtures of up to 2% of other possible impurities except CO2, the resulting H2 mass flow error is smaller than 1%. If impurities other CO2 are present, and if the molar concentration of such other impurity is below 2 mol-%, the resulting mass flow error is smaller than 1%. In the case of electrolyzer applications, where N2 and O2 are among the main impurities, even at 2 mol-% of 02, the relative Hydrogen mass flow accuracy is better than 0.25%. Hence, in a stream containing up to 2 mol-% of Oxygen, Nitrogen or a mixture of such, a measurement system maximum permissible error of <1% is achievable. Also small (e.g. <0.6%) molar percentages of H2O would not affect this accuracy much. The system is furthermore rather insensitive to small admixtures of Methane as they may occur in repurposed pipeline systems. Also in this embodiment, for increased accuracy, further analyzers, e.g. an LSA, can be added to address impurities of high concentration or of high effect on the mass flow error (e.g. CO2) in analogy to the second embodiment.

In a fourth embodiment, at least a thermal conductivity analyzer and a volume-flow meter are used. The information from the analyzer is used to:

1. Enable the calculation of mass-flow or norm volume flow based on an equation of state, also using temperature and pressure of the gas stream as recorded by a pressure and temperature measuring device. This calculation may directly output hydrogen mass flow or a total mass flow.

2. Calculate the hydrogen mass flow from the total mass flow by subtracting the mass flow of impurities as shown for the first embodiment.

In a fifth embodiment, at least a speed of sound analyzer and a volume-flow meter are used. The information from the analyzer is used to:

1. Enable the calculation of mass-flow or norm volume flow based on an equation of state, also using temperature and pressure of the stream as recorded by a pressure and temperature measuring device. This calculation may directly output hydrogen mass flow or a total mass flow. Alternatively, the speed of sound information may be provided by means of a measurement inside the volume flow meter and sent to the data processing device. This device then may use a lookup table based on the equation of state of the gas mixture (cf. Fig. 7) to determine the impurity concentration.
2. Calculate the hydrogen mass flow from the total mass flow by subtracting the mass flow of impurities as shown for the second embodiment.

Alternatively, to the mass flow, an energy flow may be calculated using a heating value based on the hydrogen and impurity concentrations.

In a sixth embodiment, a SOS gas analyzer and TCA gas analyzer and a mass flow meter report data to a data processing device, to compute the impurity-corrected hydrogen mass flow.

In a seventh embodiment, at least one laser spectroscopy-based analyzer and a flowmeter report data to a data processing device, to compute the impurity-corrected real hydrogen mass flow.

It is noted that the above embodiments are merely exemplarily, and of course other combinations of features as covered by the claimed scope are conceivable. Hence, other variations to the disclosed embodiments can be understood and effected by those skilled in the art in practicing the claimed disclosure, from the study of the drawings, the disclosure, and the appended claims. In the claims the word "comprising" or "including" does not exclude other elements or steps and the indefinite article "a" or "an" does not exclude a plurality. The mere fact that certain measures are recited in mutually different dependent claims does not indicate that a combination of these measures cannot be used to advantage.

LIST OF REFERENCE SIGNS

| 1 | data processing device |
|---|---|
| 3 | flow meter |
| 5 | gas mixture flow information |
| 7 | first compound concentration analyzer |
| 9 | first impurity concentration information |
| 10 | corrected first impurity concentration information |
| 11 | gas mixture |
| 13 | pressure sensor |
| 15 | pressure information |
| 17 | temperature sensor |
| 19 | temperature information |
| 21 | pipe |
| 23 | target gas compound flow information |
| 25 | second compound concentration analyzer |
| 27 | second impurity concentration information |
| 51 | target gas compound |
| 53 | first impurity gas compound |
| 55 | second impurity gas compound |
| 60 | computer-program product |
| 70 | computer-readable medium |
| 100 | system |
| 200 | gas delivery arrangement |
| 300 | method |
| S1-S15 | method steps |

**Claims**

1. A system (100) for determining a gas flow in a gas delivery arrangement (200), wherein the system (100) comprises:

a data processing device (1),
a flow meter (3) adapted to detect a flow of a gas mixture (11) comprising a target gas compound (51) and at least one impurity gas compound (53, 55),
wherein the flow meter (3) is adapted to provide gas mixture flow information (5) based on the detected flow to the data processing device (1),
a first compound concentration analyzer (7) calibrated to at least one first impurity gas compound (53) and adapted to detect a concentration of the first impurity gas compound (53) in the gas mixture (11),
wherein the first compound concentration analyzer (7) is adapted to provide a first impurity concentration information (9) based on the detected concentration of the first impurity gas compound (53) in the gas mixture (11) to the data processing device (1),
wherein the data processing device (1) is adapted to determine a target gas compound flow based on the gas mixture flow information (5) received from the flow meter (3) and the first impurity concentration information (9) received from the first compound concentration analyzer (7).

2.  The system (100) according to the preceding claim,
wherein the first compound concentration analyzer (7) is adapted to detect the concentration of the first impurity gas compound (53) in the gas mixture (11) at a sampling rate of less than 60 seconds, more preferably less than 30 seconds, even more preferably less than 10 seconds, and most preferably less than 1 second.

3.  The system (100) according to one of the preceding claims 1 or 2,

wherein the flow meter (3) is a mass flow meter adapted to detect a mass flow of the gas mixture (11),
wherein the mass flow meter is adapted to provide gas mixture mass flow information based on the detected mass flow to the data processing device (1), and
wherein the data processing device (1) is adapted to determine a target gas compound flow based on the gas mixture mass flow information (5) received from the mass flow meter and the first impurity concentration information (9) received from the first compound concentration analyzer (7).

4.  The system (100) according to one of the preceding claims,

wherein the flow meter (3) is a volume flow meter adapted to detect a volume flow of the gas mixture (11),
wherein the volume flow meter is adapted to provide gas mixture volume flow information based on the detected volume flow to the data processing device (1), and
wherein the data processing device (1) is adapted to determine a target gas compound flow further based on the gas mixture volume flow information received from the volume flow meter and the first impurity concentration information (9) received from the first compound concentration analyzer (7).

5.  The system (100) according to one of the preceding claims 3 or 4,
wherein the data processing device (1) is adapted to determine a calorific value of the gas mixture based on the first impurity concentration information (9).

6.  The system (100) according to one of the preceding claims,

wherein the system (100) further comprises at least one pressure sensor (13), which is adapted to determine at least one pressure of the gas mixture (11),
wherein the pressure sensor (13) is adapted to provide pressure information (15) based on the detected pressure to the data processing device (1),
wherein the data processing device (1) is adapted to determine the target gas compound flow further based on the received pressure information (15), and/or
wherein the system (100) further comprises at least one temperature sensor (17), which is adapted to determine at least one temperature of the gas mixture (11),
wherein the temperature sensor (17) is adapted to provide temperature information (19) based on the detected temperature to the data processing device (1),
wherein the data processing device (1) is adapted to determine the target gas compound flow further based on the received temperature information (19).

7.  The system (100) according to one of the preceding claims,
wherein the system (100) further comprises:

a second compound concentration analyzer (25) calibrated to at least one second impurity gas compound (55) and adapted to detect a concentration of the second impurity gas compound (55) in the gas mixture (11),
wherein the second compound concentration analyzer (25) is adapted to provide a second impurity concentration information (27) based on the detected concentration of the second impurity gas compound (55) in the gas mixture (11) to the data processing device (1),

wherein the data processing device (1) is adapted to determine the target gas compound flow further based on the second impurity concentration information (27) received from the second compound concentration analyzer (25).

8. The system (100) according to the preceding claim,
wherein the second compound concentration analyzer (25) is adapted to detect the concentration of the second impurity gas compound (55) in the gas mixture (11) at a sampling rate of less than 60 seconds, more preferably less than 30 seconds, even more preferably less than 10 seconds, and most preferably less than 1 second.

9. The system (100) according to one of the preceding claims,
wherein the first compound concentration analyzer (7) and/or the second compound concentration analyzer (25) includes one or more of an optical absorption analyzer, a thermal conductivity analyzer or a speed of sound gas analyzer.

10. The system (100) according to one of the preceding claims 7 to 9,

wherein the second compound concentration analyzer (25) is adapted to provide the second impurity concentration information (27) to the first compound concentration analyzer (7),
wherein the first compound concentration analyzer (7) is adapted to use further calibration data based on the second impurity concentration information (27) received from the second compound concentration analyzer (25),
wherein the first compound concentration analyzer (7) is adapted to provide a corrected first impurity concentration information (10) based on the first impurity concentration and the second impurity concentration information (27) to the data processing device (1),
wherein the data processing device (1) is adapted to determine a target gas compound flow based on the gas mixture flow information (5) received from the flow meter (3) and the corrected first impurity concentration information (10) received from the first compound concentration analyzer (7).

11. A method (300) for determining a gas flow in a gas delivery arrangement (200), wherein the method (300) comprises the steps of:

detecting (S1), by a flow meter (3), a flow of a gas mixture (11) comprising a target gas compound (51) and at least one impurity gas compound (53, 55),
providing (S2) gas mixture flow information (5) based on the detected flow to a data processing device (1),
calibrating (S3) a first compound concentration analyzer (7) to at least one first impurity gas compound (53),
detecting (S4), by the first compound concentration analyzer (7), a concentration of the first impurity gas compound (53) in the gas mixture (11),
providing (S5), by the first compound concentration analyzer (7), a first impurity concentration information based on the detected concentration of the first impurity gas compound (53) in the gas mixture (11) to the data processing device (1),
determining (S6), by the data processing device (1), a target gas compound flow based on the gas mixture flow information (5) received from the flow meter (3) and the first impurity concentration information received from the first compound concentration analyzer (7).

12. The method (300) according to the preceding claim,
further comprising the steps of:

calibrating (S7) a second compound concentration analyzer (25) to at least one second impurity gas compound (55),
detecting (S8) a concentration of the second impurity gas compound (55) in the gas mixture (11),
providing (S9), by the second compound concentration analyzer (25), a second impurity concentration information (27) based on the detected concentration of the second impurity gas compound (55) in the gas mixture (11) to the data processing device (1),
determining (S10), by the data processing device (1), the target gas compound flow further based on the second impurity concentration information (27) received from the second compound concentration analyzer (25).

13. The method (300) according to the preceding claim,
further comprising the steps of:

providing (S11), by the second compound concentration analyzer (25), the second impurity concentration information (27) to the first compound concentration analyzer (7),

using (S12) further calibration data for the first compound concentration analyzer (7) based on the second impurity concentration information (27) received from the second compound concentration analyzer (25),

providing (S13), by the first compound concentration analyzer (7), a corrected first impurity concentration information (10) based on the first impurity concentration and the second impurity concentration information (27) to the data processing device (1), and

determining (S14), by the data processing device (1), a target gas compound flow based on the gas mixture flow information (5) received from the flow meter (3) and the corrected first impurity concentration information (10) received from the first compound concentration analyzer (7).

14. A computer-program product (60) comprising instructions, which, when executed by a data processing device (1), cause the data processing device (1) to carry out and/or control at least partly the method (300) of any of claims 11 to 13.

15. A computer-readable medium (70) comprising instructions which, when executed by a data processing device (1), cause the data processing device (1) to carry out and/or control at least partly the method (300) of any of claims 11 to 13.

Fig. 1

Fig. 2

Fig. 3A          Fig. 3B          Fig. 3C

Fig. 4B

Fig. 4A

Fig. 5B

Fig. 5A

Fig. 6B

Fig. 6A

Fig. 7

Europäisches
Patentamt

European
Patent Office

Office européen
des brevets

# EUROPEAN SEARCH REPORT

**Application Number**

EP 24 20 8318

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 01/61285 A1 (MICRO MOTION INC [US]) 23 August 2001 (2001-08-23) * figure 1 * * page 6, line 3 - line 22 * | 1-15 | INV. G01F1/74 G01N25/00 G01N33/00 |
| | ----- | | |
| X | US 2014/119400 A1 (KINKADE BRIAN [US] ET AL) 1 May 2014 (2014-05-01) * figure 2 * * paragraph [0023] * | 1-15 | |
| | ----- | | |
| X | US 10 101 186 B2 (MEMS AG [CH]) 16 October 2018 (2018-10-16) * figure 4 * | 1-15 | |
| | ----- | | |

**TECHNICAL FIELDS SEARCHED (IPC)**

G01F
G01N

The present search report has been drawn up for all claims

| Place of search | Date of completion of the search | Examiner |
|---|---|---|
| The Hague | 11 March 2025 | Quertemont, Eric |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 8318

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2025

| Patent document cited in search report | | Publication date | Patent family member(s) | | Publication date |
|---|---|---|---|---|---|
| WO 0161285 | A1 | 23-08-2001 | AR | 027333 A1 | 26-03-2003 |
| | | | AT | E355509 T1 | 15-03-2006 |
| | | | AU | 3645101 A | 27-08-2001 |
| | | | AU | 2001236451 B2 | 08-07-2004 |
| | | | BR | 0108286 A | 29-10-2002 |
| | | | CA | 2398119 A1 | 23-08-2001 |
| | | | CN | 1401071 A | 05-03-2003 |
| | | | DE | 60126916 T2 | 28-06-2007 |
| | | | DK | 1255967 T3 | 18-06-2007 |
| | | | EP | 1255967 A1 | 13-11-2002 |
| | | | HK | 1052967 A1 | 03-10-2003 |
| | | | JP | 4338927 B2 | 07-10-2009 |
| | | | JP | 2003523508 A | 05-08-2003 |
| | | | KR | 20020086544 A | 18-11-2002 |
| | | | MX | PA02007830 A | 10-02-2003 |
| | | | MY | 133962 A | 30-11-2007 |
| | | | PL | 356440 A1 | 28-06-2004 |
| | | | US | 6612186 B1 | 02-09-2003 |
| | | | WO | 0161285 A1 | 23-08-2001 |
| US 2014119400 | A1 | 01-05-2014 | CN | 103776800 A | 07-05-2014 |
| | | | EP | 2725355 A1 | 30-04-2014 |
| | | | KR | 20140052902 A | 07-05-2014 |
| | | | US | 2014119400 A1 | 01-05-2014 |
| US 10101186 | B2 | 16-10-2018 | CN | 105606786 A | 25-05-2016 |
| | | | EP | 3021117 A1 | 18-05-2016 |
| | | | ES | 2771798 T3 | 07-07-2020 |
| | | | HK | 1221017 A1 | 19-05-2017 |
| | | | JP | 6517668 B2 | 22-05-2019 |
| | | | JP | 2016095308 A | 26-05-2016 |
| | | | RU | 2015148670 A | 15-05-2017 |
| | | | US | 2016138951 A1 | 19-05-2016 |

EPO FORM P0459

For more details about this annex : see Official Journal of the European Patent Office, No. 12/82